# EUROPEAN PATENT SPECIFICATION

(11) **EP 1 707 635 B1**
(45) Date of publication and mention of the grant of the patent: **14.11.2007**
(21) Application number: 06004462.5
(22) Date of filing: 06.03.2006
(51) Int. Cl.: C12Q 1/00, G01N 27/327

(54) **Electrode substrate, detection divice having the substrate, kit having the detection device, and detection method using the kit**
Elektrodensubstrat, Detektionsvorrichtung mit dem Substrat, Kit mit der Detektionsvorrichtung und Detektionsverfahren unter Verwendung des Kits
Substrat pour électrode, dispositif de détection comprenant ledit substrat, kit comprenant le dispositif de détection et procédé de détection employant le kit

(30) Priority: 07.03.2005 JP 2005063126
(43) Date of publication of application: 04.10.2006
(73) Proprietor: Seiko Epson Corporation, Shinjuku-ku, Tokyo 163-0811 (JP)
(72) Inventor: Fukushima, Hitoshi, Seiko Epson Corporation, Suwa-shi, Nagano-ken 392-8502 (JP); Yokokawa, Shinobu, Seiko Epson Corporation, Suwa-shi, Nagano-ken 392-8502 (JP)
(74) Representative: HOFFMANN EITLE

(56) References cited:
- WO-A-03/051506
- GB-A- 2 313 912
- US-B1- 6 214 205
- US-B1- 6 479 240
- BU H ET AL: "MODIFICATION OF FERROCENE-CONTAINING REDOX GEL SENSOR PERFORMANCE BY COPOLYMERIZATION OF CHARGED MONOMERS" ANALYTICAL CHEMISTRY, AMERICAN CHEMICAL SOCIETY. COLUMBUS, US, vol. 68, no. 22, 15 November 1996 (1996-11-15), pages 3951-3957, XP000634990 ISSN: 0003-2700

## Description

### BACKGROUND

### 1. Technical Field

The present invention relates to an electrode substrate for detecting a target material and a detection device having such a substrate, which are used in various fields. To be more specific, the invention relates to an electrode substrate that detects the transfer of carriers such as electrons, etc. to/from a target material, as well as a detection device, etc. having such a substrate.

### 2. Related Art

Since the mapping of the human genome has finished, a detection device that can efficiently and precisely identify biomolecules such as deoxyribonucleic acid (DNA), protein and antibody molecules has been playing an important role. The detection device can detect the information about the structure, function, weight, electric property and optical property of the sample containing the biomolecules and can transmit the information as data. As such detection device, for example, there is a biochip that can analyze a mass of samples in a short period of time. United States Patent No. 5445934 is a first example of related art, and United States Patent No. 6280590 is a second example of related art. The first example describes that the biochip adopts a method to measure fluorescence intensity for detecting DNA hybridization. The second example describes that the biochip adopts a method to measure a difference in DNA displacement that varies depending on the applied-electric field. Monitoring the intensity variation of the fluorescent reaction is becoming a mainstream method in this field as described in the examples.

Further, the demand for sensors or microsensor chips that are capable of a real-time in vitro, not in vivo, detection of the vital reaction wherein biomolecules such as enzymes, DNA, antibodies, etc. are concerned has been becoming remarkably high. Especially after the completion of human genome analysis, the weight has been shifted to the function analysis of genome DNA strands. In particular, the function analysis of proteins including enzymes, antibodies, etc. that are configured of DNA strands and the optimization of the target of drug development based on the functions of the proteins will be weighing more heavily. For an efficient progress of the analysis, or a high-throughput analysis, the use of DNA chips and protein chips will become more important. The core of such chip technologies is the performance of biointerfaces (hereinafter simply referred to as "BI") that serve as a detection mechanism between detection methods (optical detection using fluorescence, etc., electrochemical detection, microweight detection, etc.) and biomolecular reaction.

BI requires the capability of amplifying only the useful parameters that are well selected from the information on vital reaction and transferring the parameters after converting the parameters into detection parameters.

As a typical detection device having the BI function, electrochemical detection devices using enzyme molecules, including the marketed products of such devices, are expected to be highly demanded in the future. To be more specific, in a detection device for monitoring a high blood-sugar level caused by diabetes, real-time detection can be achieved by: immobilizing the enzyme molecules of glucose oxidase or glucose dehydrogenase, which oxidize glucose molecules into gluconic acid, onto an electrode substrate; oxidizing the glucose in the blood into glucose acid within an enzyme molecular membrane on the substrate; and capturing and detecting the oxidation current, resulting from the foregoing steps, using the electrode (refer to a third to a fifth examples of related art described later).

As described above, in a detection device for monitoring blood-sugar level, biomolecules such as enzyme molecules, etc. are generally dispersed within a soluble polymer such as cellulose, etc. to form a mixed dispersion membrane on an electrode by means of spin coating, etc. Alternatively, in another monitoring method, a pseudo vital reaction is initiated on a solid surface after putting biomolecules into an immobilized or quasi-immobilized (loosely fixed by means of noncovalent bonding) state on the surface of an electrode substrate using a self-assembled monolayer (hereinafter simply referred to as "SAM"). So far, biomolecule immobilization using a SAM has been rapidly becoming the mainstream.

U.S. Patent No. 5,445,934 is a first example of related art.

U.S. Patent No. 6,280,590 is a second example of related art.

Japanese Unexamined Patent Publication No. 6-78791 is a third example of related art.

Japanese Unexamined Patent Publication No. 6-90754 is a fourth example of related art.

Japanese Unexamined Patent Publication No. 8-505123 is a fifth example of related art.

In the above method wherein a SAM is immobilized on the surface of an electrode substrate, however, several problems have been noted as follows: (1) The monolayer makes it difficult to control the interaction between the surface of an electrode substrate and biomolecules. For example, when a biomolecule contacts a metal surface, the biomolecule, especially an enzyme etc., is denaturalized to possibly deactivate enzyme activity. (2) The control of nonspecific adsorption between the surface of an electrode substrate and biomolecules is difficult. For example, there is a possibility of adsorption between the electrode substrate surface and biomolecules, etc. due to electrostatic force or intermolecular force. (3) Since the monolayer is very thin, it is difficult to distinguish whether the target of monitoring is the oxidation current generated in enzyme reaction or the leakage current. (4) Since the above-described SAM is highly insulative, the oxidation-reduction current of enzymes, etc. cannot be detected by the electrode substrate provided under the membrane. To the contrary, the density and thickness of the SAM is so small as to easily allow the flow of leakage current, etc., which makes it difficult to form a selectively permeable membrane.

### SUMMARY

An advantage of the invention is to provide an electrode substrate having a unique membrane that allows a selective and efficient permeation of electrons.

After a diligent examination of a membrane that can allow an efficient and selective permeation of electrons considering the above circumstances, the inventor has gained a perspective for achieving the above purpose by providing a membrane having a specific configuration on an electrode substrate and completed the invention.

That is, according to a first aspect of the invention, an electrode substrate includes: an electrode; and a membrane that is provided on the electrode and has a configuration of -A-B in the order from the electrode. In the electrode substrate, the A includes an alkylene group or an alkyleneoxy group and the B includes a chain of the repeating unit of a group expressed by a chemical formula (1) below, where: X is any of a hydrogen atom, a halogen atom, or an alkyl group; and R1 represents choline phosphate or -(CH₂CH₂O)₁OH, with the 1 representing an integer of 2 or larger.

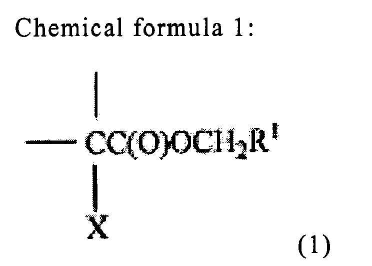

With the above configuration, the R1 having choline phosphate or -(CH₂CH₂O)₁OH enables an efficient and selective permeation of electrons for the purpose of achieving an electron transfer function. As a result, electrons are captured by the electrode.

In the electrode substrate according to the first aspect of the invention, it is preferable that the alkylene group or the alkyleneoxy group is -CH₂- or -CH₂CH₂O-. With the inclusion of -CH₂- or - CH₂CH₂O- in the A, the adhesion of biomolecules, etc. to the electrode can be prevented.

In the electrode substrate according to the first aspect of the invention, it is preferable that the B further includes a mediator or a biomolecule through the intermediary of the R1. With the inclusion of a mediator or a biomolecule in the B, the presence of a mediator or a biomolecule on the electrode substrate is ensured, which enables a prompt reaction with a target material.

In the electrode substrate according to the first aspect of the invention, it is preferable that the membrane further includes, through the intermediary of the B, a repeating unit W of a group that is expressed by a chemical formula (2) below, where: X is any of a hydrogen atom, a halogen atom, or an alkyl group; and R2 represents choline phosphate or -(CH₂CH₂O)₁OH, with the 1 representing an integer of 2 or larger.

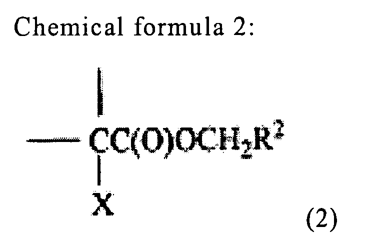

With the above configuration including choline phosphate or -(CH₂CH₂O)₁OH having an electron transfer function, an efficient and selective permeation of electrons can be achieved.

In the electrode substrate according to the first aspect of the invention, it is preferable that the W further includes a mediator or a biomolecule through the intermediary of the R2. With the above configuration, the presence of more number of mediators or biomolecules on the electrode substrate through the intermediary of the W is ensured, which enables a prompt reaction with a target material.

In the electrode substrate according to the first aspect of the invention, it is preferable that the electrode and the A are bonded through the intermediary of a sulfur atom or an oxygen atom. With the use of such a derivative, the membrane can be immobilized on the electrode substrate tightly and in an orderly direction.

In the electrode substrate according to the first aspect of the invention, it is preferable that the biomolecule is selected from a group including a nucleic acid, an enzyme, and an antibody. With the use of the above molecule, the detection of a current generated by the electron transfer based on the interaction with a target material can be achieved.

According to a second aspect of the invention, a method for forming a membrane on an electrode includes: (1) dipping the electrode into a solution containing one or more species selected from a group including a thiol halide derivative, a disulfide halide derivative, and a silanol halide derivative so that a monolayer containing an alkyl halide derivative is formed on the electrode; and (2) initiating a reaction between the alkyl halide derivative and a group expressed by a chemical formula (3) below in the presence of an inert gas. In the above method, Z is a hydrogen atom or an alkyl group; and R3 represents choline phosphate or -(CH₂CH₂O)₁OH, with the 1 representing an integer of 2 or larger.

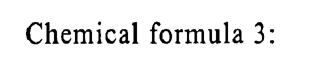

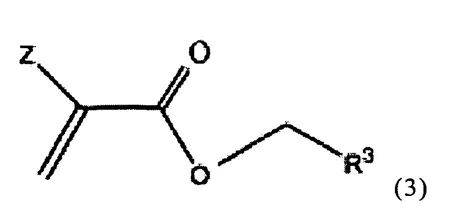

With the above method, choline phosphate or - (CH₂CH₂O)₁OH having an electron transfer function can be immobilized on the electrode.

In the method according to the second aspect of the invention, it is preferable that the method further includes (3) introducing a biomolecule or a mediator through the intermediary of: a hydroxyl group of the R3; or a maleimide group or an N-hydroxysuccinimide group that are derived from the hydroxyl group. By employing the above step, a biomolecule or a mediator can be immobilized on the electrode and, at the same time, a prompt reaction with a target material can also be achieved.

In the method according to the second aspect of the invention, it is preferable that the method further includes (4) initiating a further reaction of a group expressed by a chemical formula (4) below in the presence of an inert gas. In the above method, Z is a hydrogen atom or an alkyl group; and R4 represents choline phosphate or -(CH₂CH₂O)₁OH, with the 1 representing an integer of 2 or larger.

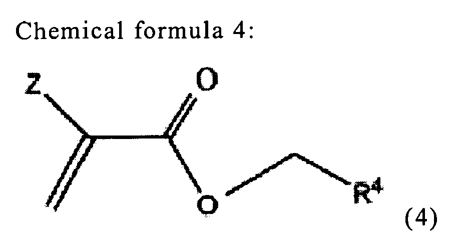

By the above method, choline phosphate or -(CH₂CH₂O)₁OH playing a role of an electron transfer function can further be provided on the electrode.

In the method according to the second aspect of the invention, it is preferable that the method further includes (5) introducing a biomolecule or a mediator through the intermediary of: a hydroxyl group of the R4; or a maleimide group or an N-hydroxysuccinimide group that are derived from the hydroxyl group. By employing the above step, another biomolecule or mediator can further be immobilized on the electrode for the purpose of more efficient detection.

In the method according to the second aspect of the invention, it is preferable that (2) and (4) are performed by means of atom transfer radical polymerization. By the above method, a membrane containing choline phosphate or -(CH₂CH₂O)₁OH having an electron transfer function can be formed controllably.

According to a third aspect of the invention, a detection device includes: the above-described electrode substrate; a counter electrode that corresponds to the electrode substrate; and a reference electrode. With the use of the above detection device, a real-time in vitro detection of a target material can be achieved.

In the detection device according to the third aspect of the invention, it is preferable that the detection device further includes a detection circuit that is electrically coupled to each of the electrode substrate, the counter electrode, and the reference electrode. With the use of the above detection circuit, the detection of a generated current can be achieved.

According to a fourth aspect of the invention, a kit for detecting a target material includes: the above-described detection device; and a biomolecule and a mediator that react with the target material. With the use of the above kit, a simple distinction of the presence of a target material can be achieved. In the kit according to the fourth aspect of the invention, it is preferable that the biomolecule is an enzyme. With the use of an enzyme, antigen-antibody reaction or oxidation-reduction reaction is initiated in conjunction with the molecules contained in a sample. Then, the electron transfer caused in such a reaction can be detected as an oxidation current or a reduction current by the electrode substrate.

In the kit according to the fourth aspect of the invention, it is preferable that the kit further includes a buffer solution. With the use of a buffer solution, the variation in pH of a sample containing a target material can be controlled, which enables a reproducible detection.

Further, according to a fifth aspect of the invention, a method for detecting a target material contained in a sample includes: preparing the above-described kit; and bringing the sample into contact with the kit. By the above method, a simple detection of a specific target material contained in a sample can be achieved.

In the method according to the fifth aspect of the invention, it is preferable that the method further includes measuring a current in the presence of the target material. Based on the measured current, the presence of a target material contained in a sample can be detected. Further, based on the comparison with a current in the absence of the target material, the concentration of the material can also be calculated.

With the electrode substrate according to the first aspect of the invention, an electrode substrate that allows a selective and efficient permeation of electrons, avoiding the adhesion of biomolecules, etc. on the surface of the electrode substrate, can be provided. Further, by the method for forming a membrane according to the second aspect of the invention, a membrane having specific molecules can be formed on an electrode by orderly controlling specific molecules. Furthermore, with the use of the above electrode substrate, a detection device that enables an easy detection of a target material can be achieved. In addition, by combining the detection device with biomolecules and mediators, a detection kit and a detection method that can achieve an easy detection of materials such as glucose, superoxide radicals, etc. can be provided.

### BRIEF DESCRIPTION OF THE DRAWINGS

The invention will be described with reference to the accompanying drawings, wherein like numbers reference like elements.

Fig. 1 shows a schematic cross section of an electrode substrate according to the first aspect of the invention.

Fig. 2 shows a schematic cross section of a first embodiment of the electrode substrate according to the first aspect of the invention.

Fig. 3 shows a schematic cross section of a second embodiment of the electrode substrate according to the first aspect of the invention.

Fig. 4 shows a schematic diagram of each step in a method for forming a membrane on an electrode according to the second aspect of the invention.

Fig. 5 is a diagram for schematically describing a specific example of manufacturing the electrode substrate according to the first aspect of the invention that is shown as a step S10 and a step S12 in Fig. 4.

Fig. 6 is a schematic diagram of glucose concentration measurement using the electrode substrate according to the first aspect of the invention.

Figs. 7A and 7B are schematic diagrams for describing the detection of superoxide radicals using the electrode substrate according to the first aspect of the invention.

Fig. 8 is a schematic plan view of a detection device according to the third aspect of the invention that has: the electrode substrate according to the first aspect of the invention; a counter electrode corresponding to the electrode substrate; and a reference electrode.

Fig. 9 is a schematic plan view of a device having a plurality of the detection devices according to the third aspect of the invention and a detection circuit which is electrically coupled to each of the detection devices.

Fig. 10 is a schematic perspective view of a system wherein the device shown in Fig. 9 is coupled to a personal computer.

### DESCRIPTION OF EXEMPLARY EMBODIMENTS

Embodiments of the invention will now be described in detail with reference to the accompanied drawings. The following embodiments are only examples for describing the invention and the invention is not limited to the following embodiments. The invention can be put into practice in various ways within the scope of the invention.

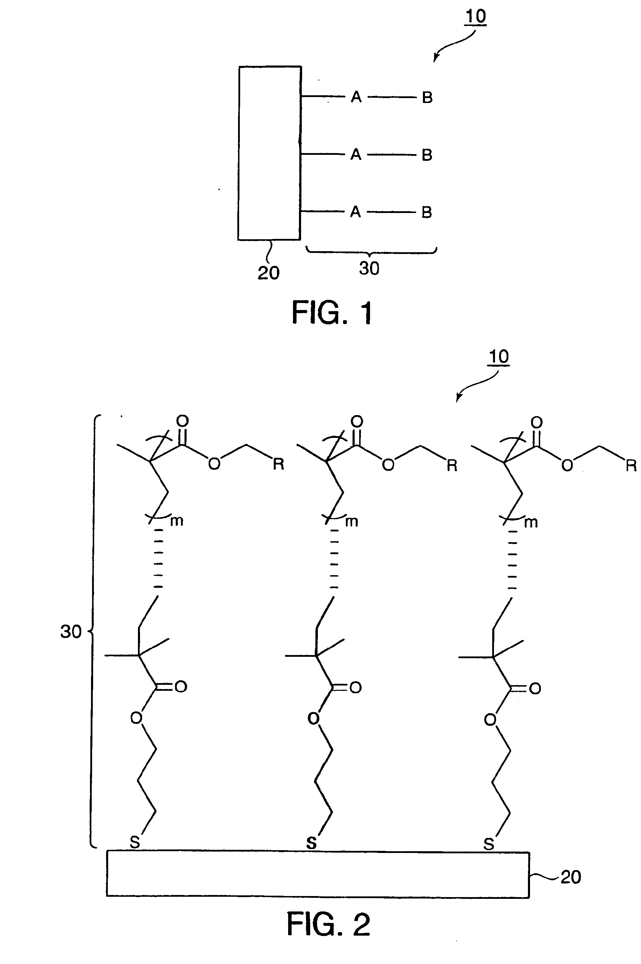

Fig. 1 shows a schematic cross section of an electrode substrate 10 according the first aspect of to the invention. The electrode substrate 10 according to the first aspect of the invention has an electrode 20 and a membrane 30 that is provided on the electrode 20. The material of the electrode 20 used in the invention is not limited to but includes carbon, gold, silver, platinum, copper, etc. The shape of the electrode, which is flat in Fig. 1, is not limited to but can be the shape of a column, etc. such as a pin shape.

The membrane 30 used in the first aspect of the invention has at least a configuration of -A-B, wherein the A includes an alkylene group or an alkyleneoxy group and the B includes a chain of the repeating unit of a group expressed by a chemical formula (1) below, where X is any of a hydrogen atom, a halogen atom, or an alkyl group.

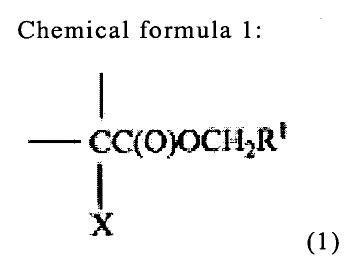

As the "halogen atom" used in the first aspect of the invention, a fluorine atom, a chlorine atom, a bromine atom, an iodine atom, etc. can be named. As the "alkyl group" used in the first aspect of the invention, a straight-chained or branched C1-C6 alkyl group having 1 to 6 carbon atoms is preferable. To be more specific, a methyl group, an ethyl group, an n-propyl group, an iso-propyl group, an n-butyl group, an iso-butyl group, a sec-butyl group, a tert-butyl group, etc. can be named. Among the foregoing, a methyl group, an ethyl group, an n-propyl group, etc. are more preferable. The R1 used in the first aspect of the invention is choline phosphate or - (CH₂CH₂O)₁OH having an electron transfer function, where the 1 is an integer of 2 or larger and 10 or lower. Further, as the "alkylene group" used in the first aspect of the invention, a C1-C4 alkylene group is preferable. To be more specific, a methylene group, an ethylene group, a methylethylene group, a trimethylene group, etc. can be named. Among the foregoing, a methylene group, an ethylene group, etc. are more preferable. Furthermore, the "alkyleneoxy group" used in the first aspect of the invention is a group configured of the "alkylene group" with an oxygen atom attached. To be more specific, a methyleneoxy group, an ethyleneoxy group, a methylethyleneoxy group, a trimethyleneoxy group, etc. can be named. Among the foregoing, a methyleneoxy group, an ethyleneoxy group, etc. are more preferable.

Fig. 2 shows a schematic cross section of a first embodiment of the electrode substrate according to the first aspect of the invention. In Fig. 2, the part A shown in Fig. 1 contains trimethylene and the part B contains a group expressed in a chemical formula (5) below, where m represents an integer of 4 to 100.

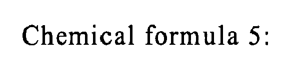

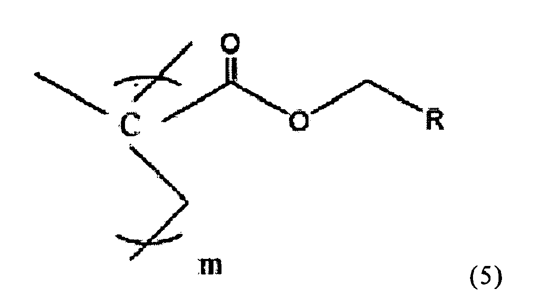

If the m is smaller than 4, biomolecules, etc. adhere on the electrode surface, which makes it difficult to measure the accurate current value. If the m is larger than 100, the transfer of carriers such as electrons, holes, etc. to/from the electrode substrate becomes less efficient. In addition, by controlling the number of m's, the thickness of the membrane 30 used in the first aspect of the invention can be controlled. In the above circumstances, the R represents choline phosphate or -(CH₂CH₂O)₁OH having an electron transfer function, where the 1 is an integer of 2 or larger and 50 or smaller. If the 1 is smaller than 2, the electron transfer function does not fully work. If the I is larger than 50, the order of the membrane 30 according to the first aspect of the invention is disturbed.

As shown in Fig. 2, between the surface of the electrode 20 and the membrane 30 according to the first aspect of the invention, a functional group bondable with the surface of the electrode 20 is provided. The functional group can be selected in accordance with the type, etc. of the electrode to be used. The part A used in the first aspect of the invention contains the functional group bondable with an electrode. As examples of the functional group used in the first aspect of the invention, a sulfur atom, which is a chalcogen atom, or a silicon atom, etc. can be named. As examples of a sulfur atom that affects bonding, compounds containing, for example, a thiol group (-SH), a disulfide group (-SS-), etc. can be named. As examples of an oxygen atom that affects bonding, a silyl group (-Si-), etc. can be named.

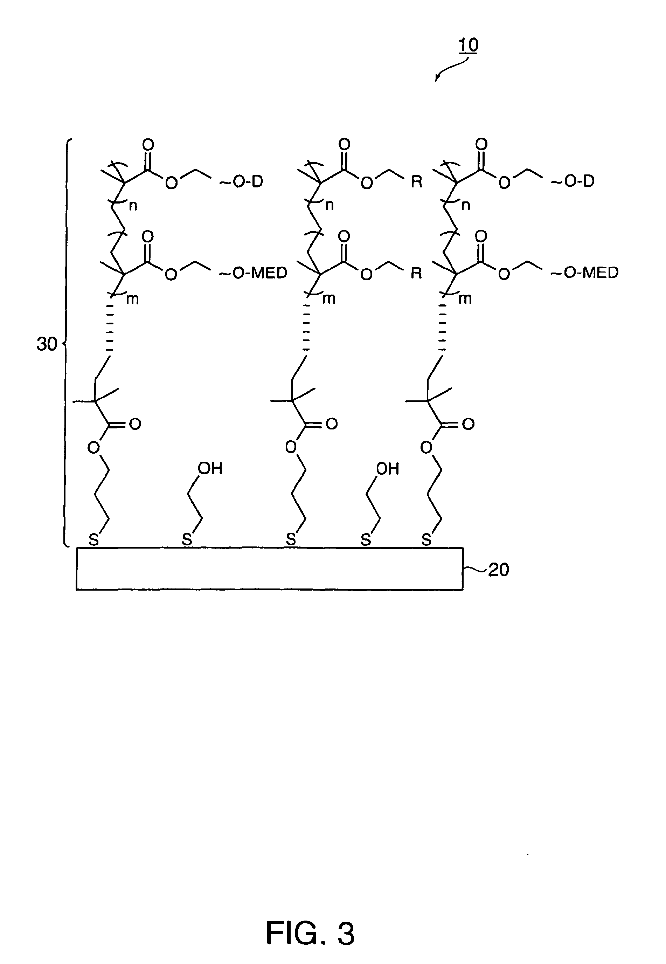

Fig. 3 shows a schematic cross section of a second embodiment of the electrode substrate 10 according to the first aspect of the invention. The electrode substrate shown in Fig. 3, basically the same as the electrode substrate shown in Fig. 2, has the electrode 20 and the membrane 30 that is configured of: the A, which is provided on the electrode 20 and contains trimethylene; and the B, which contains the above-described chemical formula (3). Further, in the membrane 30 shown in Fig. 3, a mediator (also simply called "MED") is introduced near the electrode 20 through the intermediary of: a hydroxyl group contained in the R, which is the side chain of the chemical formula (3); or a maleimide group or an N-hydroxysuccinimide group derived from the hydroxyl group, triggered by the reaction with an amino group, a thiol group, etc. that are contained in the mediator. In addition, near the surface of the membrane 30 included in the electrode substrate 10 shown in Fig. 3, a biomolecule D is introduced through the intermediary of: a hydroxyl group contained in the R, which is the side chain of the chemical formula (3); or a maleimide group or an N-hydroxysuccinimide group derived from the hydroxyl group, triggered by the reaction with an amino group, etc. that is contained in the biomolecule D. The biomolecule used in the first aspect of the invention includes nucleic acids such as DNA, RNA, etc. and proteins such as enzymes, antibodies, etc. In addition, a method for introducing the mediator MED near the surface of the electrode 20 and, at the same time, introducing the biomolecule D near the surface of the membrane 30 will be described later.

With the configuration shown in Fig. 3, the reaction between a target material contained in a sample and the biomolecule D is selectively initiated near the surface of the membrane 30. Then, the electron transfer resulting from the foregoing reaction is initiated efficiently through the intermediary of the mediator MED provided within the membrane 30 and near the electrode 20. Thus, the oxidation current or the reduction current can be measured by the electrode 20. Further, as shown in Fig. 3, by finishing the surface of the electrode 20 using hydroxyethylenethiol, etc., the direct contact between biomolecules, etc. and the electrode 20 can be avoided, which prevents the adhesion of unnecessary materials to the electrode 20. In addition, even in the case of using the electrode substrate 10 shown in Fig. 2, the electrode substrate 10 shown in Fig. 2 can serve as a sensor for a target material by adding a biomolecule such as an enzyme, etc. or a mediator into a sample containing the target material, in which the electrode substrate 10 is inserted.

The mediator used in the first aspect of the invention is a functional molecule that assists the electron transfer resulting from the interaction between a target material contained in a sample and biomolecules. Examples of the mediator used in the first aspect of the invention are not limited to but include ferrocene, a ferrocene derivative, etc. in the case of glucose concentration measurement, as described later.

The mediator used in the invention is a functional molecule that assists the electron transfer resulting from the interaction between a target material contained in a sample and biomolecules. Examples of the mediator used in the invention are not limited to but include ferrocene, a ferrocene derivative, etc. in the case of glucose concentration measurement, as described later.

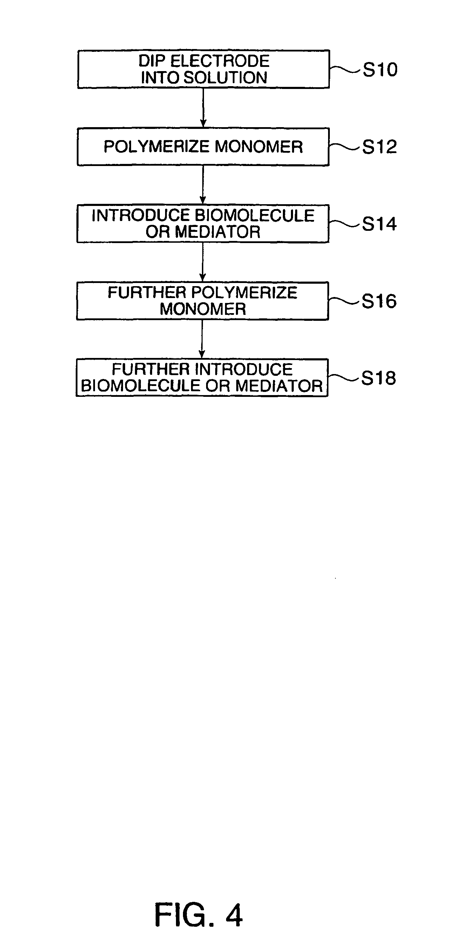

Fig. 4 shows a schematic diagram of each step in a method for forming a membrane on an electrode according to the second aspect of the invention. First, as shown in a step 10 in Fig. 4, an electrode having the surface cleansed is dipped in a solution having a functional molecule for forming a SAM. In the above step, the functional molecule for forming a SAM is a molecule that is selected from a group including a thiol halide derivative, a disulfide halide derivative, and a silanol halide derivative. Examples of the functional molecule used in the second aspect of the invention are not limited to but include, in view of polymerization reaction to be performed later, ε-mercaptoundecyl bromoisobutyrate, etc. By the above dipping process, a SAM containing an alkyl halide derivative can be formed on the electrode. Next, under an atmosphere wherein oxygen is excluded from the reaction system using an inert gas such as nitrogen, etc., (meta)acrylate monomer that is expressed as a chemical formula (3) below is polymerized in the presence of a catalyst (refer to a step S12).

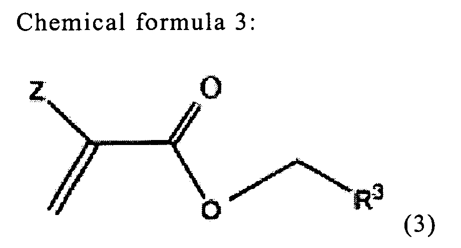

In the above formula, the Z is a hydrogen atom or an alkyl group; and the R3 represents choline phosphate or -(CH₂CH₂O)₁OH, with the 1 representing an integer of 2 or larger. The polymerization in the step S12 can be performed by means of atom transfer radical polymerization, which is described in D.M. Jones et al, Langmuir 2002, 18, 1265; and H. Ma. et al, Adv. Mater. 2004, 16, 338. The catalyst used in the polymerization is not limited to but includes a catalyst containing CuCl (monovalent), CuBr (bivalent), and bipyridine; etc.

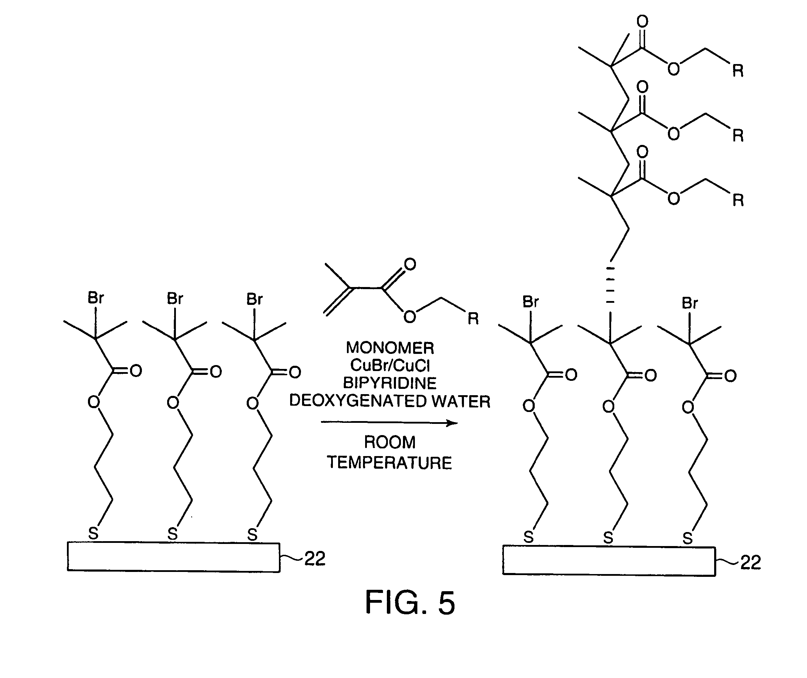

Fig. 5 is a diagram for schematically describing a specific example of manufacturing the electrode substrate 10 according to the first aspect of the invention that is shown as steps S10 and S12 in Fig. 4. A gold electrode 22 having the surface cleansed is dipped in a solution containing ε-mercaptoundecyl bromoisobutyrate to form a SAM on the surface of the gold electrode 22. After putting the gold electrode 22 having a SAM formed as above into a shrink tube, a monomer molecule that is deoxygenated by substituting oxygen with nitrogen (refer to Fig. 5: a monomer that is expressed in the chemical formula (3), where the Z is a methyl group), CuCI(monovalent), CuBr (bivalent), and bipyridine are put into the same reaction tube with an addition of deionized water, which is also deoxygenated, and evened. Further, after excluding oxygen by means of vacuum drying, etc., atom transfer radical polymerization reaction is initiated under room temperature. In addition, membrane thickness can be controlled by varying the reaction time. Thus, a first polyethylene glycol chain or a first choline phosphate group with the side chain having an electron transfer function can be introduced.

By the above method, the electrode substrate 10 wherein the membrane 30 is formed on the electrode 20 can be manufactured, as shown in Fig. 5. After the above step, a mediator and/or a biomolecule such as an enzyme or an antibody, etc. can be introduced into the membrane used in the first aspect of the invention, according to need. The method for introducing a mediator or a biomolecule will be described below.

By performing the steps to S12 in Fig. 4, the membrane 30 having a polyethylene glycol chain or a choline phosphate group in the side chain, shown in Fig. 5, is formed on the electrode. The side chain has a hydroxyl group at the end. Therefore, the hydroxyl group is converted into a maleimide group or an N-hydroxysuccinimide group that reacts with an active amino group or a thiol group contained in a biomolecule or a mediator, etc. After that, by initiating the reaction with a biomolecule and/or a mediator having an active amino group or a thiol group, the biomolecule such as an enzyme, etc. and/or the mediator can be introduced into the side chain (refer to a step S 14 in Fig. 4). Further, depending on the functional group contained in the biomolecule or the mediator, the introduction of a biomolecule or a mediator into the membrane 30 can also be performed using the hydroxyl group at the end of the side chain.

After introducing a biomolecule or a mediator, by reinitiating polymerization using an active bromine end as shown in a step S16 in Fig. 4, a second polyethylene glycol chain or a second choline phosphate group can be introduced into the side chain. Further, after converting the hydroxyl group of the second polyethylene glycol chain or the second choline phosphate group into a maleimide group or an N-hydroxysuccinimide group and then initiating the reaction with a biomolecule or a mediator having an active amino group or a thiol group, a biomolecule such as an enzyme, an antibody, etc. or a mediator can be reintroduced. In addition, for the purpose of preventing the adhesion of unnecessary materials to the electrode 20 by avoiding a direct contact between the biomolecule, etc. and the electrode 20, the surface of the electrode 20 can be finished using hydroxyethylenethiol, etc., according to need.

In the above preferable embodiments according to the second aspect of the invention, with the introduction of a mediator in the step S 14 shown in Fig. 4 and the further introduction of a biomolecule such as an enzyme, etc. in a step S 18 shown in Fig. 4, the electrode substrate 10 according to the first aspect of the invention has: a biomolecule that is provided near the surface of the membrane 30 and reacts with a target material; and a mediator that is provided near the surface of the electrode 20. By using an electrode substrate having the above configuration, a more efficient detection of a target material can be achieved. Further, the above configuration is an electrode substrate of so-called separated function type having a two-layer configuration.

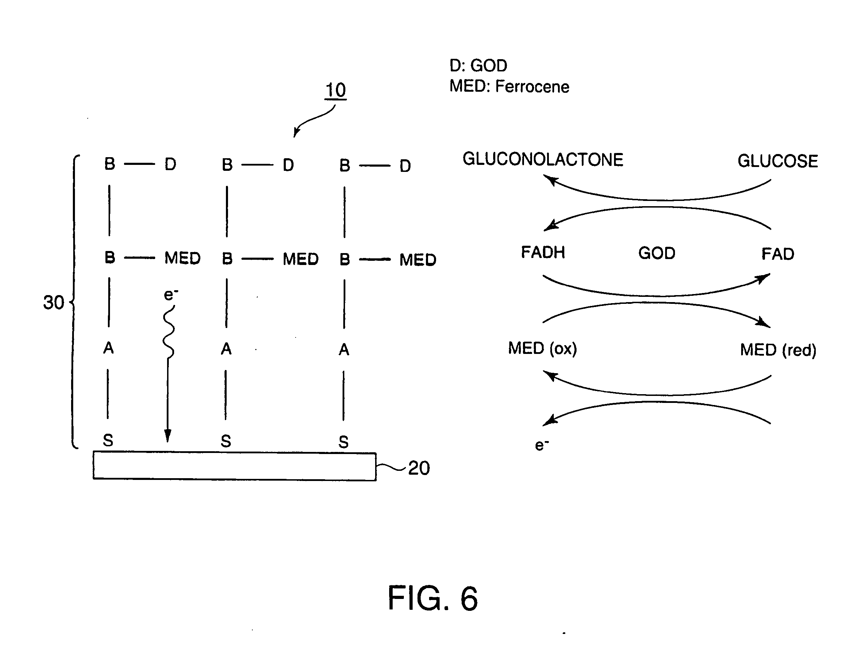

Next, a modification of the electrode substrate 10 according to the first aspect of the invention will be described. According to the first embodiment of the invention, the electrode substrate 10 can be applied to the measurement of glucose concentration (blood-sugar level) of diabetics. Fig. 6 is a schematic diagram of glucose concentration measurement using the electrode substrate 10 according to the first aspect of the invention. In Fig. 6, the detection of glucose as a target material is described, taking the case where glucose oxidase (hereinafter simply referred to as "GOD"), which is the biomolecule D, is introduced near the surface of the membrane 30 of the electrode substrate 10 according to the first aspect of the invention and, at the same time, a mediator (MED) is introduced near the surface of the electrode 20 of the membrane 30. In addition, Fig. 6, wherein the A includes trimethylene and the B includes the above-described chemical formula (5), schematically describes that the biomolecule D and the MED are introduced through the intermediary of: a hydroxyl group contained in the R in the chemical formula (5); or a maleimide group or an N-hydroxysuccinimide group derived from the hydroxyl group.

As shown in Fig. 6, in the electrode substrate 10 according to the first aspect of the invention, glucose in a blood sample is oxidized into gluconolactone by the effect of GOD and, at the same time, flavin adenine dinucleotide (FAD) in the active center of GOD, which is an enzyme, is reduced to be converted into FADH (a reductant). Then, the electrons stored in the FADH reach the electrode substrate 10 according to the first aspect of the invention through the MED. In the above sequence, the electrons selectively permeate the membrane 30 provided on the electrode substrate 10 according to the first aspect of the invention and reach the electrode 20 to be detected as an oxidation current. Therefore, the quantity of glucose as a target material in a sample can be identified in accordance with the level of measured current. In addition, current measurement can be performed by means of cyclic voltammetry, differential pulse voltammetry, etc. In Fig. 6, the case of using the electrode substrate 10 wherein an enzyme and a MED are introduced into the membrane 30 has been described. Alternatively, current detection can also be performed by adding GOD of an enzyme and a mediator MED into a sample, instead of introducing an enzyme and a MED into the membrane 30.

In the case of the glucose concentration measurement described above, examples of the mediator used in the second aspect of the invention are not limited to but include ferrocene, a ferrocene derivative, etc. The coexistence with a reactive coenzyme such as pyroquinoline quinone, nicotinamide adenine nucleotide, etc. is also allowable.

Further, the electrode substrate 10 according to the first aspect of the invention can also be applied to the detection of active oxygen species. In such a case, reactive oxygen species include superoxide anion (O₂⁻), hydroxy radicals (**·**OH), hydrogen peroxide, etc. It is known that a large intake of such reactive oxygen species into a living body causes tissue damages leading to various diseases such as inflammation, aging, carcinogenesis, myocardial infarction, etc. Especially, hydroxy radicals, which are highly active among the above reactive oxygen species, are considered to make a diffusion-controlled attack to living bodies, removing hydrogen from lipids in cells, etc. to cause inflammation and various diseases, with the lipids functioning as lipid peroxide radicals. On the other hand, living bodies have several enzymes for reducing various active oxygens. For example, superoxide dismutase (hereinafter simply referred to as "SOD") converts superoxide anion into hydrogen peroxide in disproportionation reaction. Further, catalase and glutathione peroxidase destroy hydrogen peroxide.

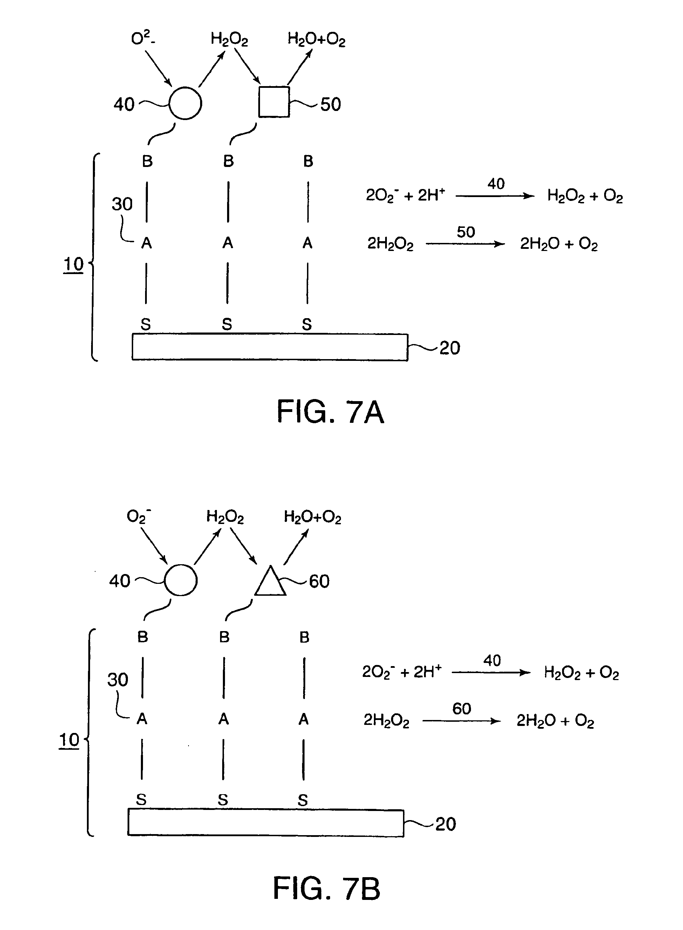

Using the above phenomena, the detection of superoxide radicals can be achieved. Figs. 7A and 7B are schematic diagrams for describing the detection of superoxide radicals using the electrode substrate 10 according to the first aspect of the invention. Fig. 7A is a schematic diagram of the electrode substrate 10 that is modified with the combination of a SOD 40 and a catalase 50. Fig. 7B is another schematic diagram of the electrode substrate 10 that is modified with the combination of the SOD 40 and a horseradish peroxidase 60. The modification of an electrode substrate using various enzymes can be performed through the intermediary of, in the case of the electrode substrate 10 having the membrane 30 according to the first aspect of the invention: a hydroxyl group at the end of the membrane 30; or a maleimide group or an N-hydroxysuccinimide group derived from the hydroxyl group. In addition, the A and B shown in Figs. 7A and 7B are the same as those in Fig. 6.

As shown in Fig. 7A, when a sample solution containing superoxide radicals is brought into contact with the electrode substrate 10 to which the SOD 40 and the catalase 50 are immobilized as enzymes, the radicals are first taken into the reactive site of the SOD enzyme and then reduced into hydrogen peroxide by the effect of the catalytic action caused by zinc, manganese, or copper atoms existing in the active site. The hydrogen peroxide is further reduced by the catalase 50, which is immobilized to the electrode substrate 10 the same as the SOD 40, and converted into water and oxygen. During the above process, enzyme-catalyzed reaction is activated by the electrons supplied from the electrode substrate 10. Therefore, the quantity of reduced electrons supplied from the electrode substrate 10 can be measured as a reduction current. The quantity of superoxide radicals contained in a sample can be identified in accordance with the level of the measured reduction current. In addition, current measurement can be performed by means of cyclic voltammetry, differential pulse voltammetry, etc.

Fig. 7B is a schematic diagram for describing the detection of superoxide radicals in the case of using the electrode substrate 10 that is modified using the horseradish peroxidase (hereinafter simply referred to as "HRP") 60 in place of the catalase 50 in Fig. 7A. In addition, the reaction mechanism of superoxide radicals in Fig. 7B is the same as that of the catalase 50 that is described in Fig. 7A. In Figs. 7A and 7B, the case where an enzyme is immobilized to the membrane 30 has been described. Alternatively, either combination of the SOD 40 and the catalase 50 or the SOD 40 and the HRP 60 can be added when a sample containing superoxide radicals, which are the target material, is brought into contact with the electrode substrate 10 according to the first aspect of the invention.

In a third embodiment of the invention shown in Figs. 7A and 7B, a mediator that assists the transfer of electrons such as ferrocene, etc. can further be included.

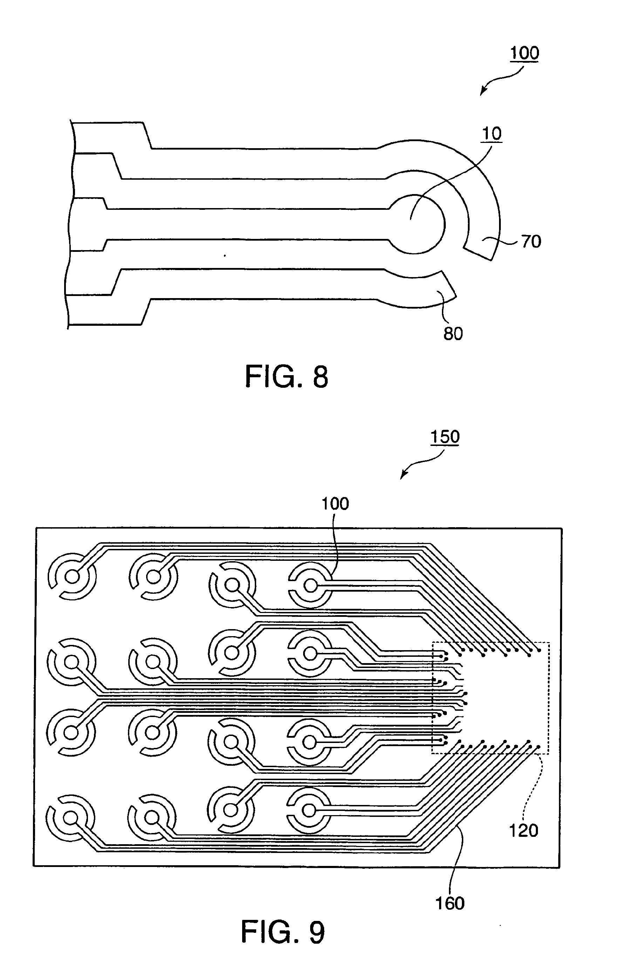

Fig. 8 is a schematic plan view of a detection device 100 according to the third aspect of the invention that has: the electrode substrate 10 according to the first aspect of the invention; a counter electrode 70 corresponding to the electrode substrate 10; and a reference electrode 80. The detection device 100 shown in Fig. 8 only includes major electrode configurations. The counter electrode 70 used in the third aspect of the invention is not limited to but configured of platinum. The reference electrode 80 used in the third aspect of the invention, which is an electrode functioning as a reference for the potentials of the electrode substrate 10 and the counter electrode 70, is not limited to but configured of silver chloride. For example, by dropping a sample containing a target material so as to cover all of the counter electrode 70; the reference electrode 80; and the electrode substrate 10 according to the third aspect of the invention to which a biomolecule that reacts with the target material is immobilized, the transfer of electrons is initiated on the electrode substrate 10 according to the third aspect of the invention. Although not illustrated in Fig. 8, by electrically coupling each of the counter electrode 70, the reference electrode 80, and the electrode substrate 10 according to the third aspect of the invention to a detection circuit 120, the detected current can be measured by the detection circuit 120. Examples of the detection circuit 120 used in the third aspect of the invention are not limited to but include a thin-film transistor, etc. In addition, current measurement can be performed by electrochemical methods such as cyclic voltammetry, differential pulse voltammetry, etc.

Further, the fourth aspect of the invention provides a detection kit having the detection device 100 according to the invention. To be more specific, the detection kit according to the fourth aspect of the invention has: the detection device 100; a biomolecule such as an enzyme, etc. that reacts with a target material; and a mediator that assists the electron transfer occurring between the target material and the biomolecule. With the detection kit according to the fourth aspect of the invention, the measurement of glucose concentration and the detection of reactive oxygen species contained in foods can be performed easily, as described in Figs. 6, 7A and 7B. Also, the detection kit according to the fourth aspect of the invention further includes a buffer solution, such as a phosphate buffer solution for example, for the purpose of controlling the pH of the reactive site, which is a water solution. In addition, by employing a thin-film configuration for the electrode substrate 10; the counter electrode 70; the reference electrode 80; and the detection circuit 120 according to the third aspect of the invention, the detection kit according to the fourth aspect of the invention can be made smaller.

Further, the fifth aspect of the invention provides a method for detecting a target material in a sample, which includes: a step for preparing the kit according to the fourth aspect of the invention; and a step for bringing the sample, such as biological samples; food samples; environment samples containing constituents in water and air, etc., containing a target material into contact with the electrode substrate 10 of the kit. Further, the presence of a target material can be observed by measuring the oxidation current or the reduction current observed by the electrode substrate 10 by means of cyclic voltammetry, differential pulse voltammetry, etc. in the presence of the target material. In addition, the concentration of the target material can also be calculated by measuring the oxidation current or the reduction current through the intermediary of the electrode substrate 10 according to the first aspect of the invention both in the presence of and in the absence of the target material after preparing a calibration curve of the target material in advance.

Fig. 9 is a schematic plan view of a device 150 having a plurality of the detection devices 100 according to the third aspect of the invention and the detection circuit 120 which is electrically coupled to each of the detection devices 100. Further, the electric coupling between the detection circuit 120 and the detection devices 100 is made by coupling the detection circuit 120 with each of the electrode substrate 10; the counter electrode 70; and the reference electrode 80 according to the third aspect of the invention, all of which are included in the detection device 100. In the case of using a thin-film transistor as the detection circuit 120, the current measured on the electrode substrate 10 can be received and further amplified on the thin-film transistor by coupling the electrode substrate 10 according to the third aspect of the invention to the drain of the thin-film transistor.

As shown in Fig. 9, with the above device, the simultaneous detection of a target material contained in the same or a plural kinds of samples can be achieved by bringing each of the detection devices 100 into contact with the same or different kinds of samples. Further, the measurement of the same kind of samples can be achieved by varying the concentration of the samples and bringing each of the samples into contact with each of the detection devices 100. In addition, the circuit for coupling each of the detection devices 100 to the detection circuit 120 is not limited to but includes silver wires 160, etc.

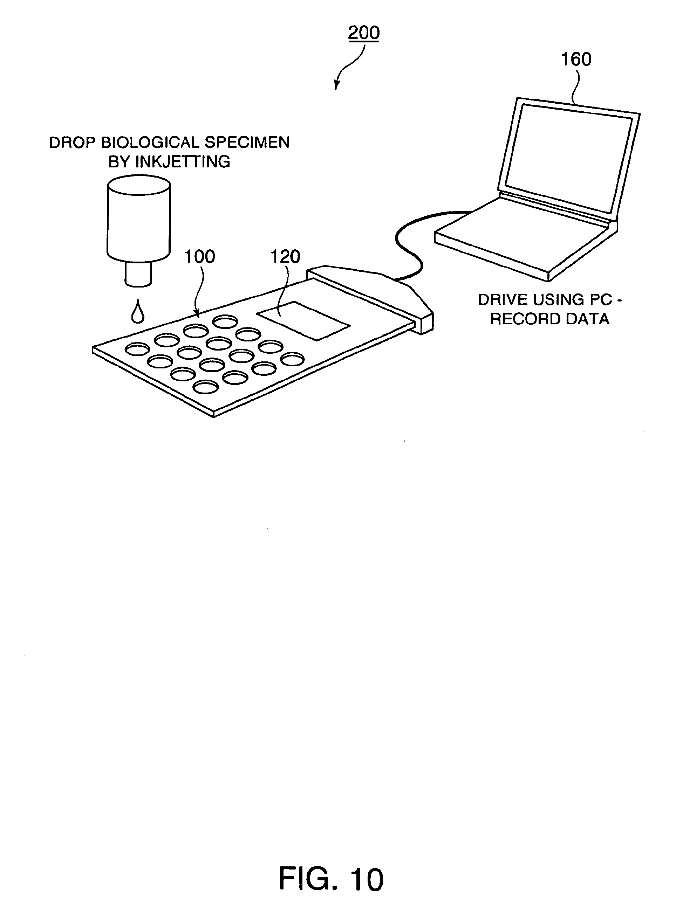

Fig. 10 is a schematic perspective view of a system 200 wherein the device 150 shown in Fig. 9 is coupled to a personal computer (hereinafter simply referred to as "PC") 160 so as to drive the device 150 using the PC 160. In addition, the device 150 is covered with, for example, a plastic substrate so as to be treated as a disposable component. Examples of the plastic substrate used in the fifth aspect of the invention are not limited to but include acrylic resin, polycarbonate resin, etc. With the above configuration, the control of blood-sugar level and the detection of reactive oxygen species contained in foods become easier, as described in Figs. 6, 7A and 7B, with only the device 150 used as a disposable component. Especially, by coupling to the PC 160, the detection circuit 120 itself can send, through the thin-film transistor, the information derived by the thin-film transistor, using an interface of USB, etc., which enables PC-driven detection. In addition, by providing the device 150 with an RF tag that is coupled to the thin-film transistor, the information derived by the thin-film transistor can be sent to the PC 160 by radio communication. Sample detection can also be performed by applying droplets of a sample to the electrode substrate 10 according to the invention by means of microspotting, inkjetting, etc. With the system 200, a sensor system 200 that is capable of real-time in vitro detection can be provided.

## Claims

1. An electrode substrate, comprising:
an electrode; and
a membrane that is provided on the electrode and has a configuration of -A-B in the order from the electrode,
wherein the A includes an alkylene group or an alkyleneoxy group and the B includes a chain of a repeating unit of a group expressed by a chemical formula (1) below, where: X is any of a hydrogen atom, a halogen atom, or an alkyl group; and R1 represents choline phosphate or - (CH₂CH₂O)ₗOH, with the 1 representing an integer of 2 or larger.

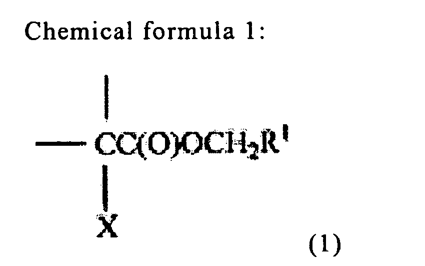

2. The electrode substrate according to Claim 1, wherein the alkylene group or the alkyleneoxy group is -CH₂- or -CH₂CH₂O-.

3. The electrode substrate according to Claim 1, wherein the B further includes a mediator or a biomolecule through an intermediary of the R1.

4. The electrode substrate according to Claim 1, wherein the membrane further includes, through an intermediary of the B, a repeating unit W of a group that is expressed by a chemical formula (2) below, where: X is any of a hydrogen atom, a halogen atom, or an alkyl group; and R2 represents choline phosphate or -(CH₂CH₂O)ₗOH, with the 1 representing an integer of 2 or larger.

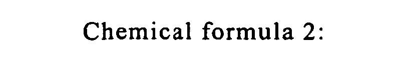

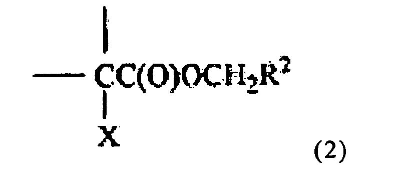

5. The electrode substrate according to Claim 1, wherein the W further includes a mediator or a biomolecule through an intermediary of the R2.

6. The electrode substrate according to Claim 1, wherein the electrode and the A are bonded through an intermediary of a sulfur atom or an oxygen atom.

7. The electrode substrate according to Claim 3, wherein the biomolecule is selected from a group including a nucleic acid, an enzyme, and an antibody.

8. A method for forming a membrane on an electrode, comprising:
(1) dipping the electrode into a solution containing one or more species selected from a group including a thiol halide derivative, a disulfide halide derivative, and a silanol halide derivative so that a monolayer containing an alkyl halide derivative is formed on the electrode; and
(2) initiating a reaction between the alkyl halide derivative and a group expressed by a chemical formula (3) below in a presence of an inert gas, where: Z is a hydrogen atom or an alkyl group; and R3 represents choline phosphate or -(CH₂CH₂O)ₗOH, with the 1 representing an integer of 2 or larger.

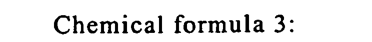

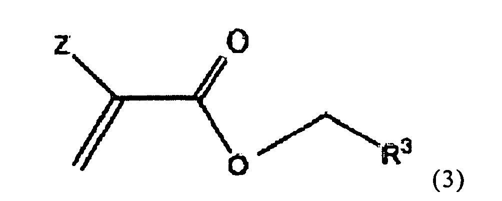

9. The method according to Claim 8, further comprising (3) introducing a biomolecule or a mediator through an intermediary of: a hydroxyl group of the R3; or a maleimide group or an N-hydroxysuccinimide group that are derived from the hydroxyl group.

10. The method according to Claim 9, further comprising (4) initiating a further reaction of a group expressed by a chemical formula (4) below in a presence of an inert gas, where: Z is a hydrogen atom or an alkyl group; and R4 represents choline phosphate or -(CH₂CH₂O)ₗOH, with the 1 representing an integer of 2 or larger.

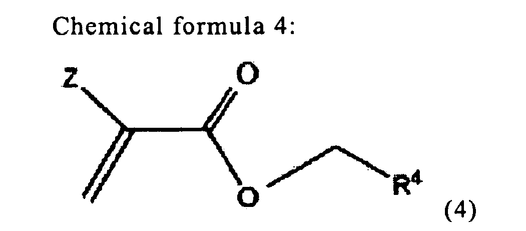

11. The method according to Claim 10, further comprising (5) introducing a biomolecule or a mediator through an intermediary of: a hydroxyl group of the R4; or a maleimide group or an N-hydroxysuccinimide group that are derived from the hydroxyl group.

12. The method according to Claim 8, wherein (2) and (4) are performed by means of atom transfer radical polymerization.

13. A detection device, comprising:
the electrode substrate according to Claim 1;
a counter electrode that corresponds to the electrode substrate; and
a reference electrode.

14. The detection device according to Claim 13, further comprising a detection circuit that is electrically coupled to each of the electrode substrate, the counter electrode, and the reference electrode.

15. A kit for detecting a target material, comprising:
the detection device according to Claim 13; and
a biomolecule and a mediator that react with the target material.

16. The kit according to Claim 15, further comprising a buffer solution.

17. A method for detecting a target material contained in a sample, comprising:
preparing the kit according to Claim 15; and
bringing the sample into contact with the kit.

18. The method according to Claim 17, further comprising measuring a current in a presence of the target material.

## Patentansprüche

1. Elektrodensubstrat, umfassend:
eine Elektrode; und
eine Membran, die auf der Elektrode bereitgestellt wird und eine Konfiguration von -A-B in der Reihenfolge von der Elektrode aufweist,
wobei A eine Alkylengruppe oder eine Alkylenoxygruppe beinhaltet, und B beinhaltet eine Kette einer sich wiederholenden Einheit einer Gruppe, ausgedrückt durch eine chemische Formel (1) unten, worin: X ein Wasserstoffatom, ein Halogenatom oder eine Alkylgrupe ist; und R¹ repräsentiert Cholinphosphat oder -(CH₂CH₂O)ₗOH, wobei 1 eine ganze Zahl von 2 oder mehr repräsentiert:

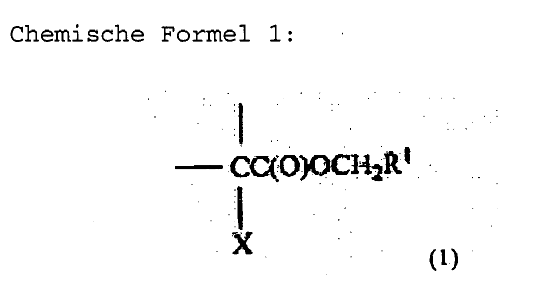

2. Elektrodensubstrat gemäß Anspruch 1, wobei die Alkylengruppe oder die Alkylenoxygruppe -CH₂- oder -CH₂CH₂O- ist.

3. Elektrodensubstrat gemäß Anspruch 1, wobei B weiterhin einen Mediator oder ein Biomolekül durch ein Intermediat von R¹ beinhaltet.

4. Elektrodensubstrat gemäß Anspruch 1, wobei die Membran weiterhin durch ein Intermediat von B eine sich wiederholende Einheit W einer Gruppe beinhaltet, die durch eine chemische Formel (2) unten ausgedrückt wird, worin: X ein Wasserstoffatom, ein Halogenatom oder eine Alkylgruppe bedeutet, und R² bedeutet Cholinphosphat oder -(CH₂CH₂O)ₗOH, wobei 1 eine ganze Zahl von 2 oder mehr repräsentiert:

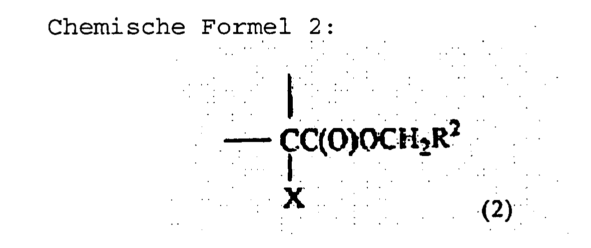

5. Elektrodensubstrat gemäß Anspruch 1, wobei W weiterhin einen Mediator oder ein Biomolekül durch ein Intermediat von R² beinhaltet.

6. Elektrodensubstrat gemäß Anspruch 1, wobei die Elektrode und A durch ein Intermediat eines Schwefelatoms oder eines Sauerstoffatoms gebunden sind.

7. Elektrodensubstrat gemäß Anspruch 3, wobei das Biomolekül gewählt ist aus der Gruppe, beinhaltend eine Nukleinsäure, ein Enzym und einen Antikörper.

8. Verfahren zur Bildung einer Membran auf einer Elektrode, umfassend:
(1) Eintauchen der Elektrode in eine Lösung, enthaltend ein oder mehr Arten, gewählt aus der Gruppe, beinhaltend ein Thiolhalogenidderivat, ein Disulfidhalogenidderivat und ein Silanolhalogenidderivat, so dass ein Monolayer, enthaltend ein Alkylhalogenidderivat auf der Elektrode gebildet wird, und
(2) Initiieren einer Reaktion zwischen dem Alkylhalogenidderivat und einer Gruppe, die durch eine chemische Formel (3) unten ausgedrückt wird in Gegenwart eines inerten Gases, worin: Z ein Wasserstoffatom oder eine Alkylgruppe ist; und R³ repräsentiert Cholinphosphat oder -(CH₂CH₂O)ₗOH, wobei 1 eine ganze Zahl von 2 oder mehr repräsentiert:

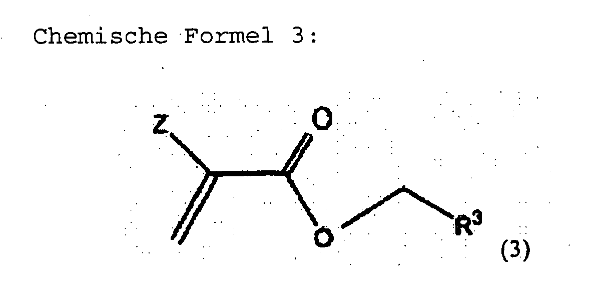

9. Verfahren gemäß Anspruch 8, weiterhin umfassend (3) das Einführen eines Biomoleküls oder eines Mediators durch ein Intermediat von: einer Hydroxylgruppe von R³; oder einer Maleimidgruppe oder einer N-Hydroxysuccinimidgruppe, die von der Hydroxylgruppe abgeleitet sind.

10. Verfahren gemäß Anspruch 9, weiterhin umfassend (4) Initiieren einer weiteren Reaktion einer Gruppe, ausgedrückt durch eine chemische Formel (4) unten in Gegenwart eines inerten Gases, worin: Z ein Wasserstoffatom oder eine Alkylgruppe ist; und R⁴ Cholinphosphat oder -(CH₂CH₂O)ₗOH repräsentiert, wobei das 1 eine ganze Zahl von 2 oder mehr repräsentiert:

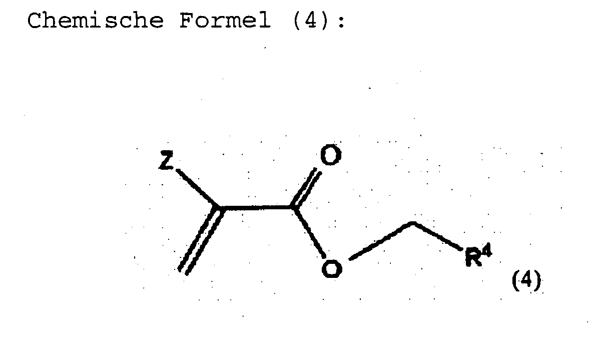

11. Verfahren gemäß Anspruch 10, weiterhin umfassend (5) Einführen eines Biomoleküls oder eines Mediators durch ein Intermediat von: einer Hydroxylgruppe von R⁴ ; oder einer Maleimidgruppe oder einer N-Hydroxysuccinimidgruppe, die von der Hydroxylgruppe abgeleitet sind.

12. Verfahren gemäß Anspruch 8, worin (2) und (4) durch eine Atomtransferradikalpolymerisation durchgeführt werden.

13. Nachweisvorrichtung, umfassend:
das Elektrodensubstrat gemäß Anspruch 1,
eine Gegenelektrode, die zu dem Elektrodensubstrat korrespondiert und
eine Referenzelektrode.

14. Nachweisvorrichtung gemäß Anspruch 13, weiterhin umfassend einen Nachweiskreislauf, der elektrisch an jeweils das Elektrodensubstrat, die Gegenelektrode und die Referenzelektrode gekoppelt ist.

15. Kit zum Nachweis eines Zielmaterials, umfassend:
die Nachweisvorrichtung gemäß Anspruch 13, und
ein Biomolekül und einen Mediator, die mit dem Zielmaterial reagieren.

16. Kit gemäß Anspruch 15, weiterhin umfassend eine Pufferlösung.

17. Verfahren zum Nachweis eines Zielmaterials., enthalten in einer Probe, umfassend:
Herstellung des Kits gemäß Anspruch 15, und
Kontaktieren der Probe mit dem Kit.

18. Verfahren gemäß Anspruch 17, weiterhin umfassend das Messen eines Stroms in Gegenwart des Zielmaterials.

## Revendications

1. Substrat d'électrode, comprenant :
une électrode ; et
une membrane qui est fournie sur l'électrode et ayant une configuration -A-B dans cet ordre depuis l'électrode,
dans lequel le A inclut un groupe alkylène ou un groupe alkylèneoxy et le B inclut une chaîne d'un motif répété d'un groupe exprimé par une formule chimique (1) ci-dessous, où : X représente indifféremment un atome d'hydrogène, un atome d'halogène ou un groupe alkyle ; et R1 représente le phosphate de choline ou - (CH₂CH₂O)ₗOH, avec le I représentant un nombre entier égal ou supérieur à 2.

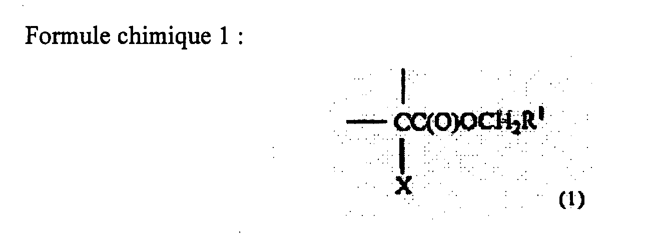

2. Substrat d'électrode selon la revendication 1, dans lequel le groupe alkylène ou le groupe alkylèneoxy est -CH₂- ou -CH₂CH₂O-.

3. Substrat d'électrode selon la revendication 1, dans lequel le B inclut en outre un médiateur ou une biomolécule par un intermédiaire du R1.

4. Substrat d'électrode selon la revendication 1, dans lequel la membrane inclue en outre, par l'intermédiaire du B, un motif répété W d'un groupe qui est exprimé par une formule chimique (2) ci-dessous, où : X représente indifféremment un atome d'hydrogène, un atome d'halogène ou un groupe alkyle ; et R2 représente le phosphate de choline ou -(CH₂CH₂O)ₗOH, avec le I représentant un nombre entier égal ou supérieur à 2.

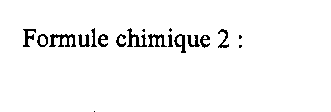

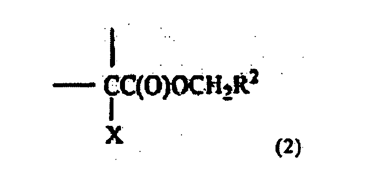

5. Substrat d'électrode selon la revendication 1, dans lequel le W inclut en outre un médiateur ou une biomolécule par un intermédiaire du R2.

6. Substrat d'électrode selon la revendication 1, dans lequel l'électrode et le A sont reliés par un intermédiaire d'un atome de soufre ou d'un atome d'oxygène.

7. Substrat d'électrode selon la revendication 3, dans lequel la biomolécule est choisie dans un groupe incluant un acide nucléique, une enzyme et un anticorps.

8. Procédé de formation d'une membrane sur une électrode, comprenant :
(1) l'immersion de l'électrode dans une solution contenant une ou plusieurs espèces choisies dans un groupe incluant un dérivé halogéné de thiol, un dérivé halogéné de disulfure et un dérivé halogéné de silanol, afin qu'une monocouche contenant un dérivé halogéné d'alkyle se forme sur l'électrode ; et
(2) l'initiation d'une réaction entre le dérivé halogéné d'alkyle et un groupe exprimé par une formule chimique (3) ci-dessous en présence d'un gaz inerte, où : Z est un atome d'hydrogène ou un groupe alkyle ; et R3 représente le phosphate de choline ou -(CH₂CH₂O)ₗOH, avec le 1 représentant un nombre entier égal ou supérieur à 2.

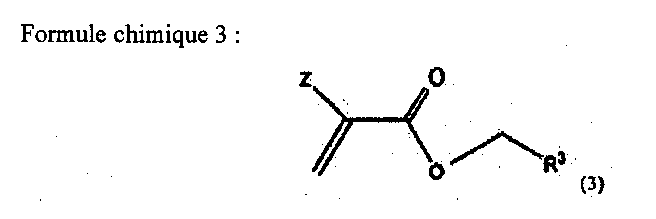

9. Procédé selon la revendication 8, comprenant en outre (3) l'introduction d'une biomolécule ou d'un médiateur par un intermédiaire : d'un groupe hydroxyle du R3 ; ou d'un groupe maléimide ou d'un groupe N-hydroxysuccinimide qui sont dérivés du groupe hydroxyle.

10. Procédé selon la revendication 9, comprenant en outre (4) l'initiation d'une réaction ultérieure d'un groupe exprimé par une formule chimique (4) ci-dessous en présence d'un gaz inerte, où : Z est un atome d'hydrogène ou un groupe alkyle ; et R4 représente le phosphate de choline or -(CH₂CH₂O)ₗOH, avec le I représentant un nombre entier égal ou supérieur à 2.

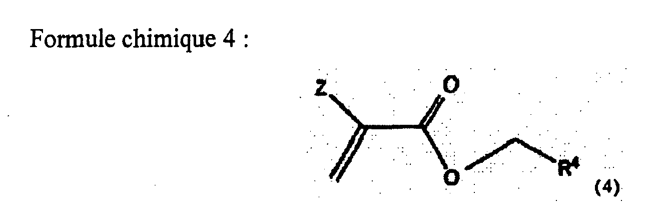

11. Procédé selon la revendication 10, comprenant en outre (5) l'introduction d'une biomolécule ou d'un médiateur par un intermédiaire : d'un groupe hydroxyle du R4 ; ou d'un groupe maléimide ou d'un groupe N-hydroxysuccinimide qui sont dérivés du groupe hydroxyle.

12. Procédé selon la revendication 8, dans lequel les étapes (2) et (4) sont réalisées au moyen d'une polymérisation radicalaire par transfert d'atome.

13. Dispositif de détection, comprenant :
le substrat d'électrode selon la revendication 1 ;
une contre-électrode qui correspond au substrat d'électrode ; et une électrode de référence.

14. Dispositif de détection selon la revendication 13, comprenant en outre un circuit de détection qui est électriquement couplé à chacun des éléments parmi le substrat d'électrode, la contre-électrode, et l'électrode de référence.

15. Kit de détection d'un matériau cible, comprenant :
le dispositif de détection selon la revendication 13 ; et
une biomolécule et un médiateur qui réagissent avec le matériau cible.

16. Kit selon la revendication 15, comprenant en outre une solution tampon.

17. Procédé de détection d'un matériau cible contenu dans un échantillon, comprenant:
la préparation du kit selon la revendication 15 ; et
la mise en contact de l'échantillon avec le kit.

18. Procédé selon la revendication 17, comprenant en outre la mesure d'un courant en présence du matériau cible.
